Europäisches Patentamt

European Patent Office

Office européen des brevets

(11) Veröffentlichungsnummer: **0 279 002**

**A1**

# EUROPÄISCHE PATENTANMELDUNG

(21) Anmeldenummer: 87102251.3

(51) Int. Cl.⁴: **A61N 5/06**

(22) Anmeldetag: **17.02.87**

Die Bezeichnung der Erfindung wurde geändert (Richtlinien für die Prüfung im EPA, A-III, 7.3).

Ein Antrag gemäss Regel 88 EPÜ auf Berichtigung von Seiten 3 und 4 der ursprünglich eingereichten Beschreibung liegt vor. Über diesen Antrag wird im Laufe des Verfahrens vor der Prüfungsabteilung eine Entscheidung getroffen werden (Richtlinien für die Prüfung im EPA, A-V, 2.2).

(43) Veröffentlichungstag der Anmeldung:
**24.08.88 Patentblatt 88/34**

(84) Benannte Vertragsstaaten:
**AT BE CH DE FR GB LI NL SE**

(71) Anmelder: **Lambertz, Christoph, Dr.**
**Auf der Sohle 31**
**D-5242 Kirchen-Sieg(DE)**

(72) Erfinder: **Lambertz, Christoph, Dr.**
**Auf der Sohle 31**
**D-5242 Kirchen-Sieg(DE)**

(74) Vertreter: **Blumbach Weser Bergen Kramer**
**Zwirner Hoffmann Patentanwälte**
**Sonnenbergerstrasse 43**
**D-6200 Wiesbaden 1(DE)**

(54) Verfahren zur Bestrahlung von Lebewesen unter Vermeidung von Schäden mit Licht, insbesondere UV-Licht, und Gerät zu seiner Durchführung.

(57) Zur Körperbestrahlung von Lebewesen mit Licht, insbesondere aus dem U.V.-Bereich ist ein Gerät (1) mit einem Polarisationsfilter (3) versehen, der zwischen der aussendenden Lichtquelle und dem zu bestrahlenden Köper angeordnet ist. Zur Vermeidung von Bestrahlungs Schädern, insbesondere der Haut, wird ein Verfahren beschrieben, bei dem das ausgesandte Licht in die Gleiche Polarisationsrichtung wie die Längspolarisation des lebenden Organismusses gebracht wird.

Fig.1

EP 0 279 002 A1

## Verfahren zur Vermeidung von Schäden, insbesondere der Haut, beim Bestrahlen von Lebewesen, wie auch des menschlichen Körpers, mit sichtbarem oder unsichtbarem, insbesondere ultraviolettem Licht

Derartige Bestrahlungen werden aus den verschiedensten Zwecken, vor allem aus kosmetischen Gründen, oft in unsachgemäßer Weise durchgeführt, so daß die Haut Schädigungen erfährt, die nachträglich zu medizinischer Behandlung Anlaß geben.

Der vorliegenden Erfindung liegt daher die Aufgabe zugrunde, Maßnahmen verfügbar zu machen, die den Gebrauch derartiger nichtmedizinischer Geräte für den Laien ermöglichen, ohne daß Schädigungen am Körper, insbesondere an der Haut, durch nicht sachgemäße Bedienung auftreten.

Die Erfindung besteht darin, daß bei der Bestrahlung mit sichtbarem oder unsichtbarem, insbesondere ultraviolettem Licht dieses in die gleiche Polarisationsrichtung wie die Längspolarisation des lebenden Organismus gebracht wird. Es hat sich gezeigt, daß bei Beachtung dieser Maßnahmen insbesondere Hautschäden bei der üblichen Bestrahlungsdauer durch Solarien oder andere Luxusbestrahlungsgeräte nicht zu beobachten waren.

In ihrer Weiterbildung schlägt die Erfindung vor, daß das auf den Körper in Längsrichtung gebrachte Licht außerdem durch einen Farbfilter gefiltert wird.

Um spezielle Anpassungen vorzunehmen, kann weiter vorgesehen werden, daß die Polarisationsebene verstellt wird.

Die Erfindung macht gleichermaßen ein Gerät zur Körperbestrahlung von Lebewesen mit Licht, insbesondere aus dem ultravioletten Bereich, verfügbar, dessen Besonderheit darin besteht, daß das Gerät mit einem Polarisationfilter versehen ist, das zwischen der aussendenden Lichtquelle und dem zu bestrahlenden Körper angeordnet ist.

Dabei erfolgt bei großen Geräten, die den Körper liegend bestrahlen, die Longitudinalausrichtung des Polarisationsfilters in Längsrichtung des Gerätes, während bei kleineren Geräten, die den ausrechten oder sitzenden Körper bestrahlen, die Ausrichtung des longitudinalen Polarisationsfilters in senkrechter Richtung erfolgt. Dabei ist es zweckmäßig, daß auf dem Gehäuse oder der die Strahlen durchdringenden Glasplatte Längsmarkierungen angebracht sind.

Mit Vorteil ist weiter vorgesehen, daß das Polarisationsfilter entsprechend zu angeordneten Markierungen einstellbar ist.

Im einzelnen zeichnet sich eine zweckmäßige Ausführungsform dadurch aus, daß das Polarisationsfilter aus einer im Strahlengang angeordneten Polarisationsfolie besteht, wobei zwischen dem Polarisationsfilter. und der Strahlungsquelle eine Farbfiltereinrichtung vorgesehen sein kann, die weiter ein Bestrahlen mit verschiedenen Farben ermöglicht.

Eine zweckmäßige Ausführungsform besteht darin, daß das Farbfilter aus einer innen auf das Polarisationsfilter geklebten Farbfolie besteht.

In ihrer Weiterbildung schlägt die Erfindung vor, daß das Gerätegehäuse aus einem Rahmenkasten besteht, über dessen Grundfläche sich eine Reflektoreinrichtung erstreckt, und daß eine parallel dazu angeordnete Deckfläche von einem Polarisationsfilter gebildet ist, wobei eine quer durch die Deckfläche gezogene Diagonale länger als der Abstand von Grund-zu Deckfläche ist.

Eine günstige Bauweise sieht vor, daß der Rahmenkasten mit einer Polarisations-Farbfilter-Kombination als Deckfläche versehen ist, wobei weiter dem Rahmenkasten im Strahlungsbereich ein zweiter Bereich zur Aufnahme von Bedienungs-und Versorgungseinrichtungen zugeordnet ist.

Die Erfindung soll nachstehend anhand von Ausführungsbeispielen unter Bezugnahme auf die beigefügten Zeichnungen näher erläutert werden.

In den Zeichnungen zeigen:

Fig. 1 die Teilansicht eines Gerätes in - schematischer Darstellung;

Fig. 2 eine Schnittansicht längs der Linie 2-2 in Fig. 1;

Fig. 3 eine Schnittansicht längs der Linie 3-3 in Fig. 1;

Fig. 4 eine Draufsicht auf ein Gerät mit Markierungen; und

Fig. 5 eine im Vergleich zur Fig. 2 abgewandelte Ausführungsform.

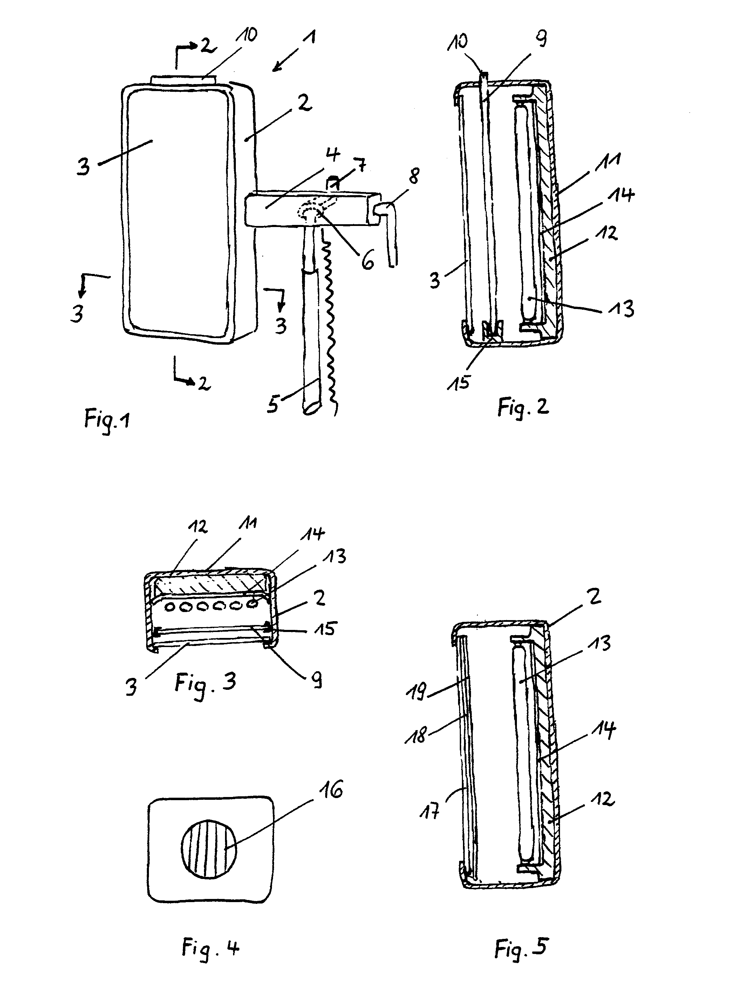

Das in Fig. 1 dargestellte Gerät 1 besteht aus einem Rahmenkasten 2, dessen Öffnung durch einen Polarisationsfilter 3 abgeschlossen ist. An dem Rahmenkasten 2 ist ein sich seitlich erstreckender Arm 4 vorgesehen, der auf einer Teleskopstange 5 ruht, die eine Höhenverstellung erlaubt. In dem Arm 4 sitzt ein Kugelgelenk 6, das eine Verstellung des Gerätes 1 ermöglicht und mit einem am Arm 4 angeordneten Stellhebel 7 eine Festlegung erlaubt. Ein Betätigungshebel 8 am Ende des Arms 7 dient zum Einschalten, gegebenenfalls mit mehreren Stufen, zum Ausschalten des Gerätes.

Wie Fig. 2 zeigt, ist hinter dem Polarisationsfilter 3 ein Farbfilter 9 angeordnet, daß mit einem Griff 10, der nur schematisch dargestellt ist und im einzelnen besonders ausgebildet sein kann, aus dem Rahmenkasten 2 entfernt werden kann und durch ein anderes Farbfilter ausgetauscht werden kann, so daß die Anwendung verschiedener Farbspektren möglich ist.

An der Rückwand 11 des Rahmenkastens 2 ist ein Sockel 12 angeordnet, indem eine Leuchtröhre 3 als Strahlungsquelle sitzt. Selbstverständlich ist auch eine jede andere Art von Strahlungsquelle denkbar. Der Sockel 12 ist mit einer reflektierenden Schicht 14 versehen, so daß die gesamte innere Rückwand des Rahmenkastens 2 als Reflektor wirkt.

In Fig. 3 ist zu sehen, wie mehrere Leuchtstoffröhren 13 in einem Rahmenkasten 2 angeordnet sein können, wobei wieder zu sehen ist, wie die Rückwand des Raumes, in dem die Leuchtröhren 3 sitzen, als Reflektor 14 ausgebildet ist, während der Raum zwischen dem Reflektor 14 und der Rückwand des Rahmenkastens 11 von dem Sockel 12 ausgefüllt wird. Das Polarisationsfilter 3 schließt den Rahmenkasten nach vorn ab, und - wie unter Bezugnahme auf Fig. 2 beschrieben wurde - sitzt dahinter ein Farbfilter 9 in Sockelschienen 15, die sein Herausnehmen bzw. Auswechseln ermöglichen.

Um das ausgesandte Licht in die gleiche Polarisationsrichtung wie die Längspolarisation des lebenden Organismus, insbesondere des menschlichen Körpers, zu bringen, ist bei großen Geräten 1, die den Körper liegend bestrahlen, die Longitudinalausrichtung des Polarisationsfilters 3 in Längsrichtung des Gerätes 1 vorgesehen, so z. B. bei den nicht dargestellten Sonnenbänken, während bei kleineren Geräten 2, die den aufrechtstehenden oder sitzenden Körper bestrahlen, wie in Fig. 1 dargestellt, die Ausrichtung des longitudinalen Polarisationsfilters 3 in senkrechter Richtung erfolgt. Bei kleineren Handgeräten, von denen Fig. 4 eine Draufsicht zeigt, sind Längsmarkierungen 60 angebracht, damit das Gerät bei der Bedienung in die richtige Lage zu der vom Kopf zum Fuß verlaufenden Längspolarisation des menschlichen Körpers gebracht werden kann. Es ist auch möglich, daß das Polarisationsfilter 3 entsprechend zu angeordneten Markierungen 16 mit Bezug auf das Gerät 1 einstellbar angeordnet ist, was in den Figuren nicht näher dargestellt ist.

Wie insbesondere aus Fig. 5 zu entnehmen ist, besteht eine einfach herzustellende Ausführungsform des Gerätes darin, daß auf einer Glasplatte 17, die den Rahmenkasten 2 des Geräts 1 nach vorn abschließt, eine Polarisationsfolie 18 auf die Innenseite geklebt wird und zur Leuchtröhre 13 zugekehrt eine weitere Farbfilterfolie 19, so daß in einfacher Weise der Rahmenkasten von einer Polarisations-Farbfilter-Kombination abgedeckt ist. Im übrigen entspricht die Ausführungsform der nach Fig. 2 beschriebenen. Bei der Dimensionierung sollte die quer durch die Deckfläche 17 gezogene Diagonale länger sein als der Abstand zur Reflektorfläche 14.

Die Erfindung hat damit ein Bestrahlungsgerät verfügbar gemacht, das von Laien zu nichtmedizinischen Zwecken aus Gründen der Kosmetik u. s. w. benutzt werden kann, ohne daß Schädigungen des Körpers, und insbesondere der Haut, zu erwarten sind.

## Ansprüche

1. Verfahren zur Vermeidung von Schäden, insbesondere der Haut, beim Bestrahlen von Lebewesen, wie auch des menschlichen Körpers, mit sichtbarem oder unsichtbarem, insbesondere ultraviolettem Licht,

**dadurch gekennzeichnet,**

daß das ausgesandte Licht in die gleiche Polarisationsrichtung wie die Längspolarisation des lebenden Organismusses gebracht wird.

2. Verfahren nach Anspruch 1, dadurch gekennzeichnet, daß das auf den Körper in Längsrichtung gebrachte Licht außerdem durch einen Farbfilter gefiltert wird.

3. Verfahren nach Anspruch 1 oder 2, dadurch gekennzeichnet, daß die Polarisationsebene verstellt wird.

4. Gerät zur Körperbestrahlung von Lebewesen mit Licht, insbesondere aus dem Ultraviolettbereich, dadurch gekennzeichnet, daß das Gerät (1) mit einem Polarisationsfilter (3) versehen ist, das zwischen der aussendenden Lichtquelle (13) und dem zu bestrahlenden Körper angeordnet ist.

5. Gerät nach Anspruch 4, dadurch gekennzeichnet, daß bei großen Geräten (1), die den Körper liegend bestrahlen, die Longitudinalausrichtung des Polarisationsfilters (3) in Längsrichtung des Geräts (1) erfolgt.

6. Gerät nach Anspruch 4, dadurch gekennzeichnet, daß bei kleineren Geräten (1), die den aufrechtstehenden oder sitzenden Körper bestrahlen, die Ausrichtung des longitudinalen Polarisationsfilters (3) in senkrechter Richtung erfolgt.

7. Gerät nach einem der Ansprüche 4 bis 6, dadurch gekennzeichnet, daß auf dem Gehäuse (2) oder der die Strahlen durchdringenden Glasplatte (3) Längsmarkierungen (16) angebracht sind.

8. Gerät nach einem der Ansprüche 4 bis 7, dadurch gekennzeichnet, daß das Polarisationsfilter (3) entsprechend zu angeordneten Markierungen (16) einstellbar ist.

9. Gerät nach einem der Ansprüche 4 bis 8, dadurch gekennzeichnet, daß das Polarisationsfilter (3) aus einer im Strahlengang angeordneten Polarisationsfolie (18) besteht.

10. Gerät nach einem der Ansprüche 4 bis 9, dadurch gekennzeichnet, daß zwischen dem Polarisationsfilter (3, 18) und der Strahlungsquelle (13) eine Farbfiltereinrichtung (9, 19) vorgesehen ist.

11. Gerät nach Anspruch 10,
dadurch gekennzeichnet, daß die Farbfiltereinrichtung (9) das Bestrahlen mit verschiedenen Farben ermöglicht.

12. Gerät nach Anspruch 10,
dadurch gekennzeichnet, daß die Farbfiltereinrichtung (9) aus einer innen auf das Polarisationsfilter (18) geklebten Farbfolie (19) besteht.

13. Gerät nach einem der Ansprüche 4 bis 12,
dadurch gekennzeichnet, daß das Gerätegehäuse aus einem Rahmenkasten besteht, über dessen Grundfläche sich eine Reflektoreinrichtung (14) erstreckt, und daß eine parallel dazu angeordnete Deckfläche von einem Polarisationsfilter (3, 18) gebildet ist, wobei eine quer durch die Deckfläche (3, 18) gezogene Diagonale länger als der Abstand von Reflektor-(14) zur Deckfläche (3, 18) ist.

14. Gerät nach Anspruch 13,
dadurch gekennzeichnet, daß der Rahmenkasten (2) mit einer Polarisations-Farbfilter-Kombination (18, 19) als Deckfläche versehen ist.

15. Gerät nach einem der Ansprüche 4 bis 14,
dadurch gekennzeichnet, daß dem Rahmenkasten (2) als Strahlungsbereich ein zweiter Bereich (4) zur Aufnahme von Bedienungs-und Versorgungseinrichtungen (6, 7, 8) zugeordnet ist.

Fig.1

Fig. 2

Fig. 3

Fig. 4

Fig. 5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | GB-A- 262 703 (HANOVIA) <br> * Insgesamt * <br><br> --- | 1,4,15 | A 61 N 5/06 |
| A | EP-A-0 190 533 (EVB) <br><br> * Seite 14, Zeilen 1-7; Seite 16, Zeilen 1-8 * <br><br> ----- | 2,10-12 | |

RECHERCHIERTE SACHGEBIETE (Int. Cl.4)

A 61 N
F 21 V

Der vorliegende Recherchenbericht wurde für alle Patentansprüche erstellt.

| Recherchenort | Abschlußdatum der Recherche | Prüfer |
|---|---|---|
| DEN HAAG | 20-10-1987 | LEMERCIER D.L.L. |

# BLUMBACH · WESER · BERGEN · KRAMER
## ZWIRNER · HOFFMANN

Patentanwälte  Sonnenberger Straße 43  6200 Wiesbaden 1

Europäisches Patentamt
Erhardtstraße 27

8000 München 2

EUROPEAN PATENT ATTORNEYS

IN WIESBADEN
P. G. BLUMBACH DIPL.-ING. PATENTANWALT
P. BERGEN PROFESSOR DR. JUR. DIPL.-ING.
G. ZWIRNER DIPL.-ING. DIPL.-W.-ING. PATENTANWALT

IN MÜNCHEN
R. KRAMER DIPL.-ING. PATENTANWALT
W. WESER DIPL.-PHYS. DR. RER. NAT. PATENTANWALT
E. HOFFMANN DIPL.-ING. PATENTANWALT

| Ihr Zeichen<br>Your Reference | Ihre Nachricht vom<br>Your Communication of | Unser Zeichen<br>Our Reference | WIESBADEN |
|---|---|---|---|
| | | Bg/ho | 30.11.87 |

Anmeldung 87 102 251.3
Dr. Christoph Lambertz

Im Nachgang zum Schreiben vom 24.11.87

werden zwei neue Beschreibungsseiten 3 und 4 überreicht, in denen die
folgenden Bezugszeichen richtiggestellt sind:
Seite 3, Zeile 35, "Leuchtröhre 13"  und
Seite 4, Zeile 26, "Längsmarkierungen 16" .

Es wird gebeten, für die Veröffentlichung die Korrekturen zu berücksichtigen.

EPA EPO-OEB
DG 1
Reçu:
0 8 -12- 1937
03 | ANL ZEICHN.

Dr. Bergen

Sonnenberger Straße 43  6200 Wiesbaden  Telefon 06121-56 00 91  Telex 4186237  Telefax     06121-56 72 09  Telegramme Patentconsult
Radeckestraße 43  8000 München 60  Telefon 089-88 36 03  Telex 5212313  Telefax 089-8 34 46 18  Telegramme Patentconsult